# EUROPEAN PATENT SPECIFICATION

(11) **EP 1 514 919 B1**
(45) Date of publication and mention of the grant of the patent: **05.12.2007**
(21) Application number: 04020246.7
(22) Date of filing: 26.08.2004
(51) Int. Cl.: C12M 1/24, C12M 1/12, C12M 3/06

(54) **Tissue culture vessel**
Behälter zur Kultivierung von Geweben
Récipient pour la culture de tissus

(30) Priority: 09.09.2003 US 501495 P; 19.08.2004 US 922679
(43) Date of publication of application: 16.03.2005
(73) Proprietor: Becton, Dickinson and Company, Franklin Lakes, NJ 07417-1880 (US)
(72) Inventor: Shanler, Michael S., Bedford, Massachusetts 01730 (US); Hughes, Kevin M., Burlington MA 01803 (US)
(74) Representative: Weber, Thomas

(56) References cited:
- EP-A- 0 337 677
- WO-A-00/23331
- WO-A-96/39533
- US-A- 5 047 347
- US-A1- 2002 039 785

## Description

### 1. Field of the Invention

The invention relates generally to tissue culture vessels. More particularly, the invention relates to vessels for growing cells, microorganisms and tissue in a culture medium and then conveniently accessing materials in the vessel.

### 2. Description of the Related Art

Tissue culture vessels are used widely in laboratories for many purposes. For example, tissue culture vessels are used to culture microorganisms or tissues in a culture medium or agar. The microorganisms or tissues are permitted to grow under controlled conditions. The tissues then may be accessed periodically and tested.

May tissue culture vessels are of generally prismatic shape with a plurality of upstanding sidewalls extending between opposed top and bottom walls. The sidewalls generally are constructed so that the length and width of the vessel exceed the height. As a result, the bottom wall of the vessel defines a fairly large surface area relative to the volume of the vessel. A tubular neck typically is formed at one of the sidewalls of the vessel to provide access to the interior. The outer surface of the neck may be formed with an array of threads for threadedly receiving a cap.

Tissue culture vessels typically are employed by removing the cap from the neck of the vessel and depositing a selected amount of a liquid growth medium in the vessel. Cells or tissue then are inserted into the vessel through the opening in the neck and the cap is replaced on the neck. Several such vessels typically are arranged in a fairly dense array and at a controlled location in a laboratory. The vessels may be accessed periodically to assess the growth of the cells or tissue in the vessel. The access to the interior of the vessel may be achieved by removing the cap from the neck of the vessel and inserting a scrapper, swab or pipette through the neck sufficiently for accessing the tissue in the growth media. This procedure is effective but very inefficient and not well suited to automated laboratory equipment.

U.S. Patent No. 4,334,028 shows a tissue culture vessel with a frangible zone formed in the top wall of the vessel. The frangible zone is defined by a region of reduced thickness that may be cut or broken to access the interior of the vessel. An area of the top wall near the frangible zone defines a hinge. Thus, the frangible zone effectively defines a trap door that can be rotated about the hinge to access the interior of the vessel.

Some tissues must be exposed to air to grow properly. U.S. Patent No. 5,047,347 shows a vessel with a gas permeable membrane incorporated into a wall of the vessel or a portion of the closure. A cover is hingedly mounted near the gas permeable membrane for selectively covering the membrane. The hinged cover for the membrane shown in U.S. Patent No. 5,047,347 is not well suited for use with automated laboratory testing equipment.

Many laboratory analyses can be completed with smaller amounts of cells or tissues and smaller volumes of growth medium. Thus, more laboratory tests can be completed within a smaller area of a laboratory. However, it is necessary to locate the smaller volume of liquid growth media and the smaller areas of cells or tissues in a predictable manner within a vessel so that the cells or tissues can be harvested easily.

Laboratory equipment is available for collecting small amounts of liquid with a robotic device. For example, multi-well plate assemblies are employed in laboratories and have an array of small wells arranged in a rectangular matrix. A typical multi-well plate may include 96 wells arranged in an 8x12 rectangular matrix. Laboratory equipment also includes robotic pipette devices for automatically entering access ports of the multi-well plate assembly for removing small amounts of liquid in the respective wells. The robotic device then moves the array of pipettes to another location so that the small amount of liquid collected on the respective pipettes can be analyzed. The above-described tissue culture vessels are not well suited for use with robotic devices, and hence are used primarily with less efficient manual procedures for growing and harvesting tissue cultures.

### SUMMARY OF THE INVENTION

It is an object of the invention to provide a tissue culture vessel that is well suited for use with robotic devices.

The tissue culture vessel of the present invention is defined by claim 1.

It comprises a top wall, a bottom wall and a plurality of sidewalls extending between the top and bottom walls. A hollow neck extends from one of the sidewalls and provides communication with the interior of the vessel. Exterior portions of the neck may include cap attachment structures, such as an array of threads. Thus, a cap may be attached removably to the neck for closing the interior of the vessel. One of the walls of the vessel spaced from the neck is formed with at least one aperture sealing septum extends across the aperture. The self-sealing septum is formed from a resealable elastomeric material and may have a slit extending at least partly through the elastomeric material. Alternatively, the septum may be formed with a cross-cut defining a generally X-shaped pair of cuts each of which extends at least partly through the septum. The septum could be configured to be accessed by a pipette tip or other pointed implement for accessing liquid and cells in the vessel.

The vessel may further include a second aperture covered by a membrane that permits a flow of gas across the membrane without permitting liquid to flow across the membrane. The membrane preferably is disposed on a surface of the vessel that will remain dry, and hence permits gas exchange, oxygenation or humidity control in the vessel.

The top and bottom walls of the vessel may define major surface areas as compared to areas defined by any of the sidewalls. Additionally the bottom wall may include footprint alignment features for positioning the vessel in a specified location and orientation to permit access by a robotic device. For example, the bottom surface may have structural features for fixing and orienting the vessel relative to alignment tiles on a robot deck. The robot then can be programmed to access the vessel at the self-sealing septum for automatically testing or harvesting the tissue or cells being grown in the vessel.

The bottom wall of the vessel can be configured to define a trough in which liquid growth medium will collect due to forces of gravity. The trough defined in the bottom wall of the vessel may be registered with the self-sealing septum.

The self-sealing septum may be configured to permit a single pipette or other collection device to pass through the septum and into an area of the vessel at which liquid media will collect. Alternatively, the self-sealing septum may permit a plurality of pipettes or other collection devices to pass simultaneously through the self-sealing septum. For example, the self-sealing septum may be elongated and may have an elongate slit or a linear array of cross-cuts. The trough defined by the bottom wall of the vessel may extend substantially along an axis defined by the elongate self-sealing septum.

The self-sealing septum and/or the membrane may be incorporated into one or more caps mounted in the apertures in the wall of the vessel. The self-sealing septum and the membrane can be mounted in the same cap.

In still other embodiments, the vessel can be configured to be stored on one of the small side surfaces. Thus, the major surfaces define sides aligned substantially vertically. In these embodiments, the self-sealing septum and the membrane may be provided in a side surface substantially opposite the surface on which the vessel will be supported.

### BRIEF DESCRIPTION OF THE DRAWINGS

FIG. 1 is an exploded perspective view of a culture vessel in accordance with the invention.

FIG. 2 is a perspective view, partly in section, of the vessel of FIG. 1 in the fully assembled condition.

FIG. 3 is a top plan view of the vessel.

FIG. 4 is s cross-sectional view taken along line 4-4 in FIG. 3.

FIG. 5 is a top plan view similar to FIG. 3, but showing a cover with an alternate septum.

FIG. 6 is an exploded perspective view of an alternate vessel in accordance with the invention.

FIG. 7 is a top plan view of a vessel similar to the vessel shown in FIG. 6, but showing an alternate septum.

FIG. 8 is an exploded view of a cap and vessel according to a further embodiment.

FIG. 9 is a perspective view of an alternate cap.

FIG. 10 is an exploded perspective view of the tissue culture vessel in proximity to a robot deck.

### DETAILED DESCRIPTION

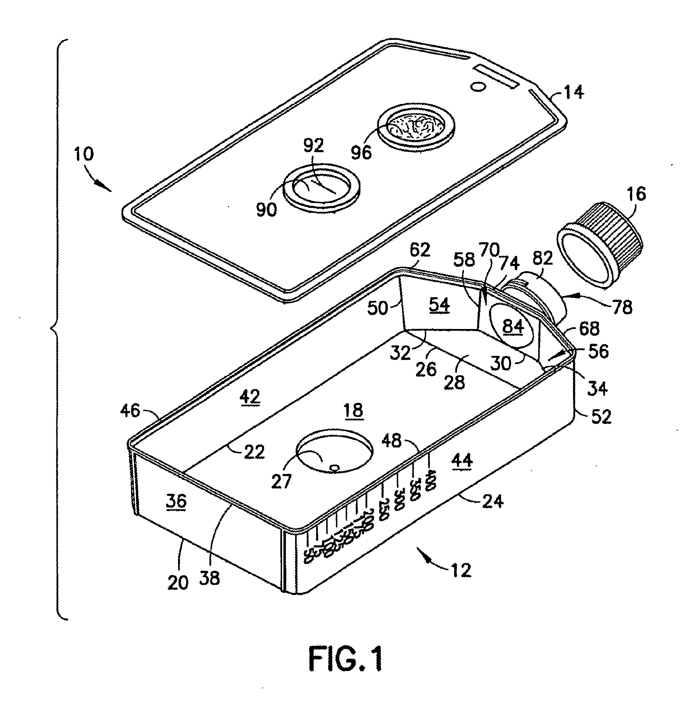

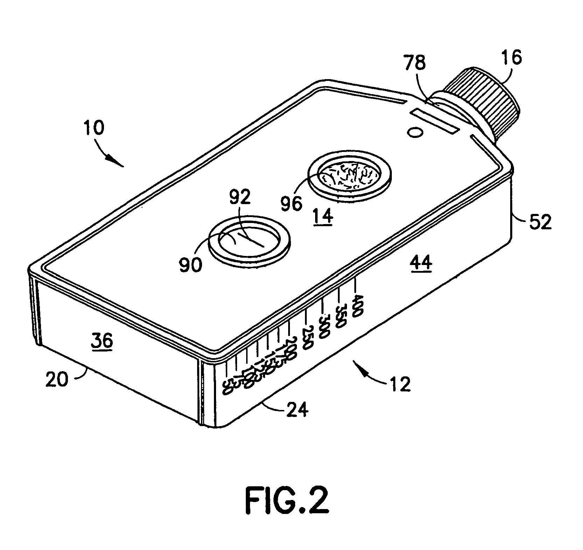

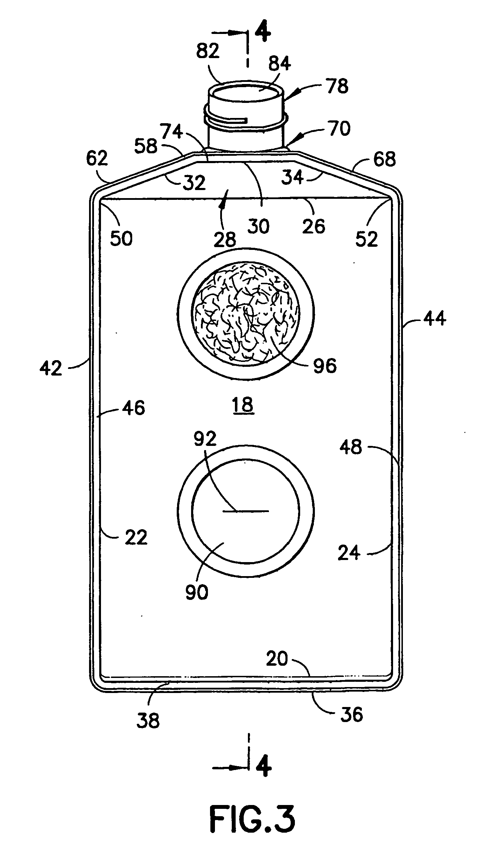

A tissue culture vessel in accordance with the invention is identified generally by the numeral **10** in FIGS. 1-4. Tissue culture vessel **10** is a generally hexagonal container with a base **12**, a cover **14** and a cap **16**. Base **12** is formed unitarily from a plastic material, and preferably a polystyrene. Base **12** includes a substantially planar rectangular bottom wall **18** with a back end **20,** first and second sides **22** and **24** and a front end **26**.

A substantially conically generated trough **27** extends down at a location on bottom wall **18** substantially centrally between back and front ends **20** and **26** and substantially centrally between sides **22** and **24.** A substantially planar isosceles trapezoidal ramp **28** extends unitarily from front end **26** of bottom wall **18** and is aligned to bottom wall **18** at an obtuse angle of about 150°. Hence, the plane of trapezoidal ramp **28** defines an incline of about 30° when bottom wall **18** is supported on a horizontal surface. Front end **26** of bottom wall **18** defines the longer of two parallel bases for trapezoidal ramp **28**. Ramp **28** further includes a shorter parallel base end **30** and first and second equal sides **32** and **34** that converge from end **26** toward end **30.**

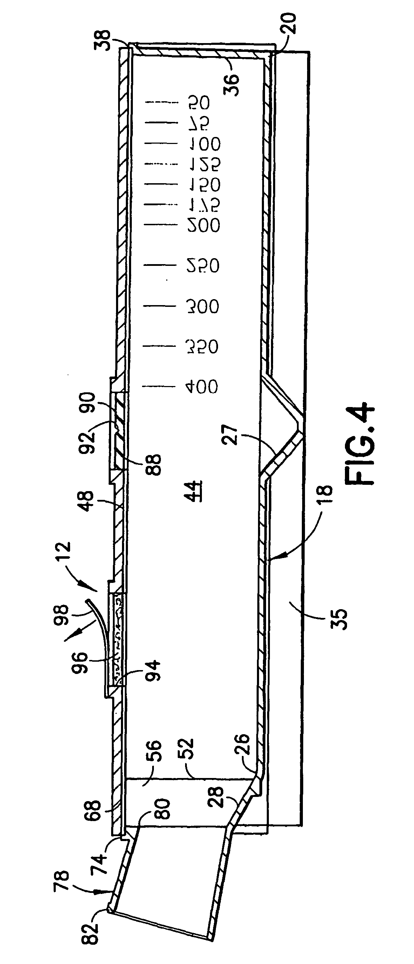

Bottom supports **35** extend down from bottom wall **18**, as shown in FIG. 4. Bottom ends of the bottom supports **35** define a plane extending substantially parallel to planar portions of bottom wall **18**. The plane defined by the bottom ends of the bottom supports **35** is coplanar with or lower than the bottom of bottom trough **27.** Bottom supports **35** also define an outer periphery substantially in the shape of a rectangle.

Base **12** of vessel **10** includes a substantially rectangular back wall **36** that projects orthogonally from bottom wall **18** at a location adjacent back end **20** of bottom wall **18**. Back wall **36** includes a top edge **38** aligned substantially parallel to bottom wall **18**. Base **12** also includes first and second substantially parallel rectangular sidewalls **42** and **44** that extend orthogonally from bottom wall **18** at locations adjacent first and second sides **22** and **24** respectively. First and second sidewalls **42** and **44** include top edges **46** and **48** respectively that are parallel to bottom wall **18** and substantially coplanar with top edge **38** of back wall **36.** Sidewalls **42** and **44** have front ends **50** and **52** substantially aligned with opposed sides of front end **26** of bottom wall **18**.

Base **12** further includes first and second substantially planar transition walls **54** and **56** that converge toward one another from front ends **50** and **52** of first and second sidewalls **42** and **44** respectively. First transition wall **54** is substantially trapezoidal and has a top edge **62** that is substantially in the plane defined by top edges **38, 46** and **48.** Second transition wall **56** also is substantially trapezoidal and includes a top edge **68** substantially in the plane defined by top edges **38, 46, 48** and **62.**

Base **12** of vessel **10** further includes a substantially planar front wall **70** aligned substantially orthogonal to the plane defined by bottom wall **18**. Front wall **70** is substantially rectangular and has first and second sides coincident with the front ends of first and second transition walls **54** and **56** respectively. Front wall **70** further includes a top edge **74** that extends between top edges **62** and **68** of first and second transition walls **54** and **56**. Top edge **74** lies in the plane defined by top edges **38, 46, 48, 62** and **68.**

Base **12** of vessel **10** further includes a generally tubular neck **78** that extends forwardly from front wall **70**. Neck **78** includes an open rear end **80** at front wall **70** that communicates with the region of base **12** above bottom wall **18** and ramp **28**. Neck **78** further includes a front end **82** and a tubular passage **84** extending between rear end **80** and front end **82**. Portions of neck **78** adjacent front end **82** are substantially cylindrically generated and exterior regions of neck **78** adjacent front end **82** include an array of external threads for threaded engagement of cap **16**.

Cover **14** of vessel **10** is substantially planar and defines a hexagon with a shape that permits cover **14** to rest on top edges **38, 46, 48, 62, 68,** and **74** of base **12** or to nest slightly with the vertical walls of base **12**. Cover **14** may be secured in position on base **12** by appropriate application of adhesive or by a known bonding technique, such as ultrasonic welding.

Cover **14** includes a septum aperture **88** at a location aligned with conically generated trough **35** in bottom wall **18,** as shown in FIG. 4. A self-sealing septum **90** is secured in septum aperture **88.** Septum **90** is formed from an elastomeric material and is provided with a longitudinal slit **92** aligned substantially along a diameter of aperture **88.** Slit **92** may extend entirely through septum **90** or partly through septum **90** and will enable an access device, such as a pipette to be passed through septum **90** for collecting a tissue culture. However, the elastomeric material of septum **90** will reseal upon removal of the access device.

Cover **14** also is formed with a membrane aperture **94** and a membrane **96** is mounted securely in membrane aperture **94,** as shown in FIG. 4. Membrane **96** is formed from a material that will permit gas exchange or oxygenation across the otherwise substantially impervious walls of vessel **10**. Membrane **96** preferably is sealed initially by a removable sealing layer **98.** Sealing layer **98** can be kept in place for those situations where gas exchange is not desired or can be removed at an appropriate time for situations where gas exchange is desired.

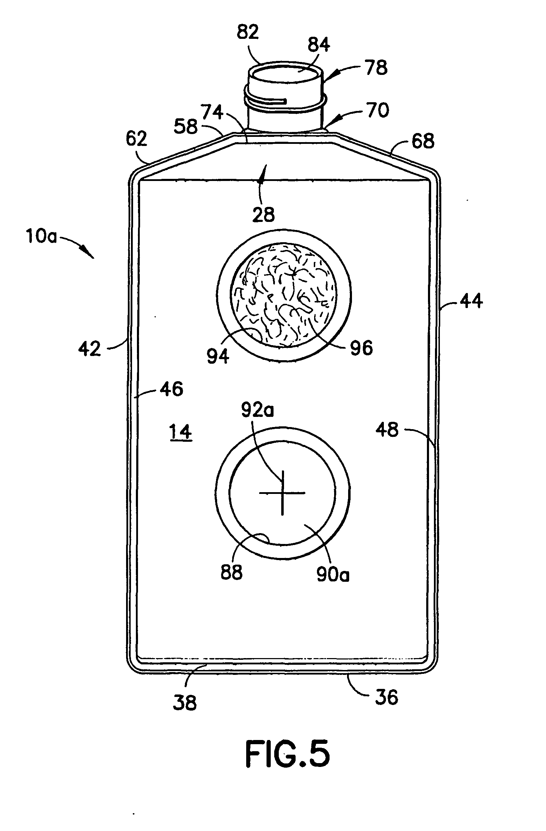

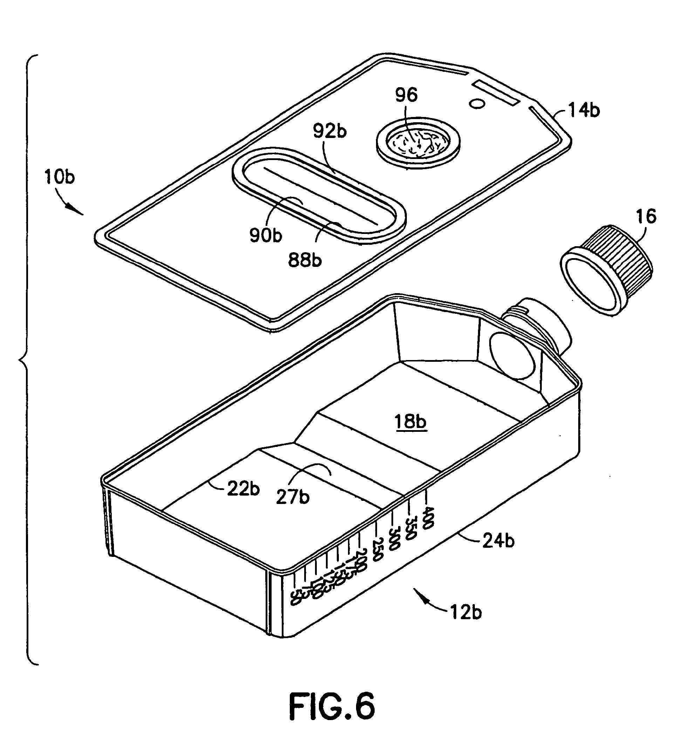

FIG. 5 shows a vessel **10a** that is substantially identical to vessel **10** described and illustrated in FIGS. 1-5. In particular, vessel **10a** includes a base **12** identical to the base **12** of vessel **10** and a cap **16** identical to the cap of vessel **10.** Vessel **10a** further includes a cover **14** that is substantially identical to the cover of vessel **10.** However, septum aperture **88** of cover **14** is provided with a septum **90a** with a cross-cut **92a** as shown in FIG. 6. Cross-cut **92a** may provide a more preferable access for certain types of access devices.

Another alternate culture vessel is identified generally by the numeral **10b** in FIG. 6. Culture vessel **10b** include a base **12b,** a cover **14b** and a cap **16.** Base **12b** is very similar to base **12** of culture vessel **10** described and illustrated above. However, bottom wall **18b** is provided with an elongate trough **27b** that extends substantially continuously between side edges **22b** and **24b.** All other aspects of base **12b** are identical to base **12**, and are not described again. Cover **14b** is very similar to cover **14**. However, cover **14b** includes an elongate generally elliptoid septum aperture **88b** and a correspondingly configured septum **90b.** Septum **90b** is provided with an elongate resealable slot **92b** disposed and aligned to register substantially with elongate trough **27b** in bottom wall **18b.** Slot **92b** enables a plurality of access devices, such as pipettes to be passed simultaneously through slot **92b** for obtaining a plurality of tissue or cell cultures simultaneously. Slot **92b** then will reseal simultaneously for access again at a later stage. All other aspects of culture vessel **10b** are substantially identical to culture vessel **10.**

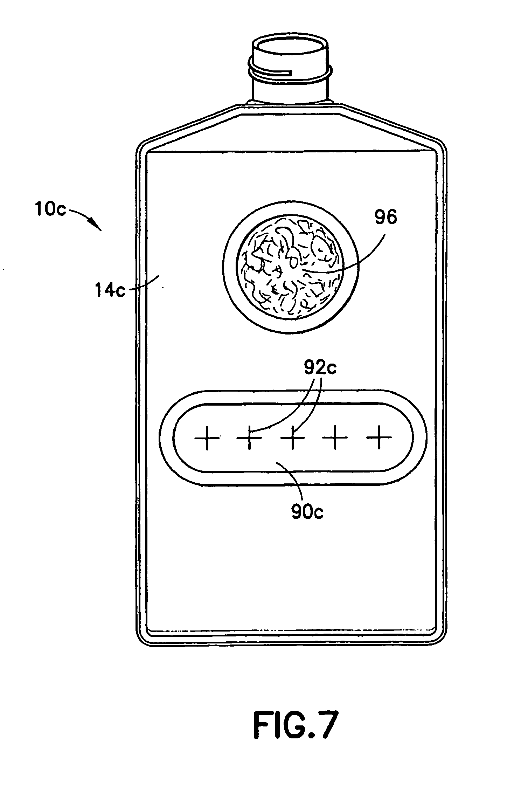

A variation of culture vessel **10b** is identified by the numeral **10c** in FIG. 7. Culture vessel **10c** includes a base and a cap substantially identical to the culture vessel **10b.** The culture vessel in FIG. 7 further includes a cover **14c** substantially identical to the cover **14b** described and illustrated with respect to FIGS. 6. However, cover **14c** includes a septum **90c** with a plurality of spaced apart cross-cuts **92c.** Each cross-cut **92c** may be substantially identical to the cross-cuts **92a** illustrated in FIG. 5. Cross-cuts **92c** may facilitate access for certain types of access devices, while enabling simultaneous access by a plurality of such devices.

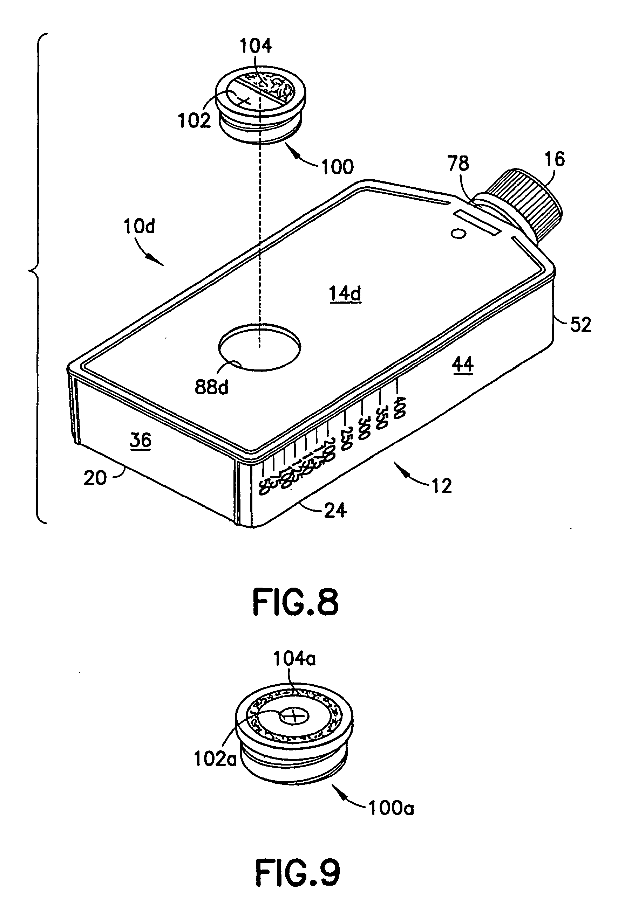

FIG. 8 shows a culture vessel **10d** similar to culture vessel **10** described and illustrated above. In particular, culture vessel **10d** includes a base **12** and a cap **16** substantially identical to the corresponding parts of the culture vessel **10** described and illustrated with respect to FIGS. 1-5. However, culture vessel **10d** has a cover **14d** with only one aperture **88d** and a threaded cap **100** is mounted in aperture **88d.** A septum **102** and a membrane **104** are mounted in cap **100.** Septum **102** and membrane **104** each are substantially semi-circular. However other shapes can be provided, such as a circular septum **102a** and an annular membrane **104a,** as shown in FIG. 9. These designs enable the type of septum (e.g., straight cut or cross-cut) to be changed for a particular application. Similarly, the type of membrane can be changed for a particular application. Alternatively, a solid cap can be engaged in aperture **88d** for those situations where no septum or no membrane is desired. Similarly, a cap with only a split septum or only a membrane can be employed.

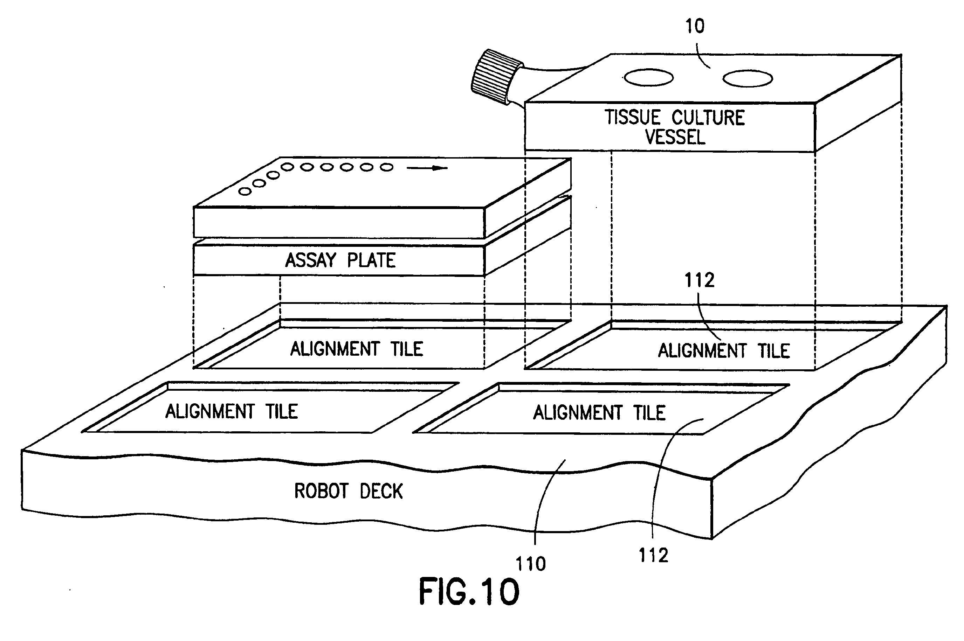

The culture vessel of the subject invention is well suited for use with automotive robotic devices for accessing the interior of the culture vessel and obtaining samples of cell or tissue cultures. For example, as shown in FIG. 10, culture vessel **10** can be used with a robot deck **110** that has a plurality of rectangular alignment tile recesses **112.** Bottom supports **35** of culture vessel **10** are dimensioned to nest in alignment tile **112** to provide a specific arrangement of X,Y coordinates for culture vessel **10**.

## Claims

1. A tissue culture vessel having a base (12) with a bottom wall (18) and a plurality of sidewalls(36,42,44,70) extending up from said bottom wall, a cover (14) extending across said sidewalls and opposed to said bottom wall(18),
**characterized in that**
the cover (14) is formed with at least one opening extending therethrough and a septum (90) extending across said aperture for permitting access to interior portions of said vessel by a medical device, said septum being formed from an elastomeric material that is resealable after access by the medical device.

2. A tissue culture vessel of Claim 1, a hollow neck (82) formed at one (70) of said sidewalls and providing communication to interior portions of said vessel for pouring a liquid media into or out of said vessel, and a closure (16) securely mounted to said neck for selectively closing said neck..

3. The tissue culture vessel of Claim 2, wherein said septum (90) is formed with at least one slit (92,92a,92b,92c) extending at least partly through said septum.

4. The tissue culture vessel of Claim 3, wherein said at least one split comprises two slits intersecting one another for defining a cross-cut in said septum.

5. The tissue culture vessel of Claim 1, wherein said aperture (88) in said cover is substantially circular.

6. The tissue culture vessel of Claim 5, wherein said bottom wall (18) of said base (12) is formed with a trough (27) substantially opposed to said aperture in said cover for collecting liquid media at a location below said septum.

7. The tissue culture vessel of Claim 1, wherein said aperture (88b) is an elongate aperture.

8. The tissue culture vessel of Claim 7, wherein said bottom wall (18b) of said base (12b) includes an elongate trough (27b) formed therein and substantially aligned with said elongate aperture (88b) in said cover for collecting liquid media in said trough at locations substantially aligned with said septum.

9. The tissue culture vessel of Claim 7, wherein said septum (90b) includes at least one slit extending at least partly through said septum for facilitating access by a medical device.

10. The tissue culture vessel of Claim 9, wherein said at least one slit comprises a plurality of pairs of intersecting slits (92c) at spaced apart locations along said septum.

11. The tissue culture vessel of Claim 1, wherein said septum (102,102a) is secured in a cap (100,100a) and wherein said cap is mounted to said aperture (88d) in said cover.

12. The tissue culture vessel of Claim 11, wherein one (70) said sidewalls of said base (12) is formed with a hollow neck (78) for pouring liquid media into or out of said vessel.

13. The tissue culture vessel of Claim 12, further comprising a closure (16) releasably mounted to said neck.

14. The tissue culture vessel of Claim 1, wherein said cover (14) further includes a membrane (96) for providing gas communication between the interior of said vessel and ambient surroundings.

15. The tissue culture vessel of Claim 14, wherein said aperture (88) is a septum aperture and wherein said cover (14) further comprises a membrane aperture, said membrane (96) being mounted to said membrane aperture.

16. The tissue culture vessel of Claim 1, further comprising a cap (100,100a) mounted to said aperture (88d) in said cover (14d), said septum (102,102a) being mounted in a portion of said cap, a second portion of said cap being formed with a membrane (104,104a) for providing gas communication to interior portions of said tissue culture vessel.

## Patentansprüche

1. Gewebekulturbehälter mit einer Basis (12), die eine Bodenwand (18) und mehrere von der Bodenwand nach oben abstehende Seitenwände (36,42,44,70) aufweist, und mit einem Deckel (14), der sich über den Seitenwänden und der Bodenwand (18) gegenüberliegend erstreckt,
**dadurch gekennzeichnet, dass**
der Deckel (14) mit mindestens einer Durchgangsöffnung und einem sich über der Öffnung erstreckenden Septum (90) versehen ist, um einer medizinischen Vorrichtung den Zugriff auf Innenbereiche des Behälters zu ermöglichen, wobei das Septum aus einem elastomeren Material ausgebildet ist, das nach dem mittels der medizinischen Vorrichtung erfolgenden Zugriff wiederabdichtbar ist.

2. Gewebekulturbehälter nach Anspruch 1, mit einem hohlen Hals (82), der an einer (70) der Seitenwände ausgebildet ist und eine Verbindung mit Innenbereichen des Behälters ermöglicht, um ein Flüssigmedium in den Behälter hinein oder aus diesem heraus zu gießen, und einem fest an dem Hals angeordneten Verschluss (16) zum selektiven Schließen des Halses.

3. Gewebekulturbehälter nach Anspruch 2, bei dem in dem Septum (90) mindestens ein Schlitz (92,92a,92b,92c) ausgebildet ist, der sich mindestens teilweise durch das Septum erstreckt.

4. Gewebekulturbehälter nach Anspruch 3, bei dem der mindestens eine Schlitz mindestens zwei Schlitze aufweist, die einander derart schneiden, dass sie einen kreuzförmigen Schnitt in dem Septum bilden.

5. Gewebekulturbehälter nach Anspruch 1, bei dem die in dem Deckel ausgebildete Öffnung (88) im Wesentlichen kreisförmig ist.

6. Gewebekulturbehälter nach Anspruch 5, bei dem in der Bodenwand (18) der Basis (12) eine der Öffnung des Deckels im Wesentlichen gegenüberliegende Mulde (27) ausgebildet ist, um Flüssigmedium an einer unterhalb des Septums gelegenen Stelle zu sammeln.

7. Gewebekulturbehälter nach Anspruch 1, bei dem die Öffnung (88b) eine längliche Öffnung ist.

8. Gewebekulturbehälter nach Anspruch 7, bei dem in der Bodenwand (18b) der Basis (12b) eine längliche Mulde (27b) ausgebildet ist, die mit der in dem Deckel vorgesehenen länglichen Öffnung (88b) im Wesentlichen ausgerichtet ist, um in der Mulde Flüssigmedium an Stellen zu sammeln, die mit dem Septum im Wesentlichen ausgerichtet sind.

9. Gewebekulturbehälter nach Anspruch 7, bei dem das Septum (90b) mindestens einen zumindest teilweise durch das Septum verlaufenden Schlitz aufweist, um einer medizinischen Vorrichtung den Zugriff zu erleichtern.

10. Gewebekulturbehälter nach Anspruch 9, bei dem der mindestens eine Schlitz mehrere Paare einander schneidender Schlitze (92c) an voneinander beabstandeten Stellen entlang des Septums aufweist.

11. Gewebekulturbehälter nach Anspruch 1, bei dem das Septum (102, 102a) in einer Kappe (100,100a) gesichert ist und bei dem die Kappe an der in dem Deckel ausgebildeten Öffnung (88d) befestigt ist.

12. Gewebekulturbehälter nach Anspruch 11, bei dem an einer (70) der Seitenwände der Basis (12) ein hohler Hals (78) ausgebildet ist, um ein Flüssigmedium in den Behälter hinein oder aus diesem heraus zu gießen.

13. Gewebekulturbehälter nach Anspruch 12, ferner mit einem lösbar an dem Hals befestigten Verschluss (16).

14. Gewebekulturbehälter nach Anspruch 1, bei dem der Deckel (14) ferner eine Membran (96) zur Ermöglichung von Gasverbindung zwischen dem Inneren des Behälters und der Umgebung aufweist.

15. Gewebekulturbehälter nach Anspruch 14, bei dem die Öffnung (88) eine Septumöffnung ist und bei dem der Deckel (14) ferner eine Membranöffnung aufweist, wobei die Membran (96) an der Membranöffnung befestigt ist.

16. Gewebekulturbehälter nach Anspruch 1, ferner mit einer Kappe (100, 100a), die an der in dem Deckel (14d) ausgebildeten Öffnung (88d) befestigt ist, wobei das Septum (102,102a) in einem Teil der Kappe angeordnet ist und wobei an einem zweiten Teil der Kappe eine Membran (104,104a) ausgebildet ist, um eine Gasverbindung mit Innenbereichen des Gewebekulturbehälters zu ermöglichen.

## Revendications

1. Récipient de culture de tissu ayant une base (12) avec une paroi inférieure (18) et une pluralité de parois latérales (36, 42, 44, 70) s'étendant vers le haut à partir de ladite paroi inférieure, un couvercle (14) s'étendant sur lesdites parois latérales et opposé à ladite paroi inférieure (18),
**caractérisé en ce que** :
le couvercle (14) est formé avec au moins une ouverture s'étendant à travers celui-ci et un septum (90) s'étendant sur ladite ouverture pour permettre l'accès aux parties intérieures dudit récipient grâce à un dispositif médical, ledit septum étant formé à partir d'un matériau élastomère qui est refermable après l'accès grâce au dispositif médical.

2. Récipient de culture de tissu selon la revendication 1, un goulot creux (82) formé au niveau de l'une (70) desdites parois latérales et fournissant la communication aux parties intérieures dudit récipient pour déverser un milieu liquide à l'intérieur ou à l'extérieur dudit récipient, et un bouchon (16) monté de manière fixe sur ledit goulot pour fermer sélectivement ledit goulot.

3. Récipient de culture de tissu selon la revendication 2, dans lequel ledit septum (90) est formé avec au moins une fente (92, 92a, 92b, 92c) s'étendant au moins partiellement à travers ledit septum.

4. Récipient de culture de tissu selon la revendication 3, dans lequel ladite au moins une fente comprend deux fentes se croisant l'une par rapport à l'autre pour définir une coupe transversale dans ledit septum.

5. Récipient de culture de tissu selon la revendication 1, dans lequel ladite ouverture (88) dans ledit couvercle est sensiblement circulaire.

6. Récipient de culture de tissu selon la revendication 5, dans lequel ladite paroi inférieure (18) de ladite base (12) est formée avec une goulotte (27) sensiblement opposée à ladite ouverture dans ledit couvercle pour collecter le milieu liquide au niveau d'un emplacement situé au-dessous dudit septum.

7. Récipient de culture de tissu selon la revendication 1, dans lequel ladite ouverture (88b) est une ouverture allongée.

8. Récipient de culture de tissu selon la revendication 7, dans lequel ladite paroi inférieure (18b) de ladite base (12b) comprend une goulotte allongée (27b) formée à l'intérieur de celle-ci et sensiblement alignée avec ladite ouverture allongée (88b) dans ledit couvercle pour collecter le milieu liquide dans ladite goulotte à des emplacements sensiblement alignés avec ledit septum.

9. Récipient de culture de tissu selon la revendication 7, dans lequel ledit septum (90b) comprend au moins une fente s'étendant au moins partiellement à travers ledit septum pour faciliter l'accès par un dispositif médical.

10. Récipient de culture de tissu selon la revendication 9, dans lequel ladite au moins une fente comprend une pluralité de paires de fentes qui se coupent (92c) à des emplacements espacés le long dudit septum.

11. Récipient de culture de tissu selon la revendication 1, dans lequel ledit septum (102, 102a) est fixé dans un capuchon (100, 100a) et dans lequel ledit capuchon est monté sur ladite ouverture (88d) dans ledit couvercle.

12. Récipient de culture de tissu selon la revendication 11, dans lequel l'une (70) desdites parois latérales de ladite base (12) est formée avec un goulot creux (78) pour déverser le milieu liquide à l'intérieur et à l'extérieur dudit récipient.

13. Récipient de culture de tissu selon la revendication 12, comprenant en outre un bouchon (16) monté de manière amovible sur ledit goulot.

14. Récipient de culture de tissu selon la revendication 1, dans lequel ledit couvercle (14) comprend en outre une membrane (96) pour fournir la communication de gaz entre l'intérieur dudit récipient et le milieu ambiant.

15. Récipient de culture de tissu selon la revendication 14, dans lequel ladite ouverture (88) est une ouverture de septum et dans lequel ledit couvercle (14) comprend en outre une ouverture de membrane, ladite membrane (96) étant montée sur ladite ouverture de membrane.

16. Récipient de culture de tissu selon la revendication 1, comprenant en outre un capuchon (100, 100a) monté sur ladite ouverture (88d) dans ledit couvercle (14d), ledit septum (102, 102a) étant monté dans une partie dudit capuchon, une seconde partie dudit capuchon étant formée avec une membrane (104, 104a) pour fournir la communication de gaz aux parties intérieures dudit récipient de culture de tissu.
